(19) Europäisches Patentamt
European Patent Office
Office européen des brevets

(11) Publication number: **0 077 197**
**B1**

# (12) EUROPEAN PATENT SPECIFICATION

(45) Date of publication of patent specification: **28.05.86**

(21) Application number: **82305376.4**

(22) Date of filing: **08.10.82**

(51) Int. Cl.$^4$: **A 61 K 31/505,** A 61 K 9/06, A 61 K 31/20 // (A61K31/505, 31:20)

## (54) Use of methotrexate and/or retinoic acid for the manufacture of compositions for use in preventing proliferation of remnant lens epithelial cells after extracapsular extraction.

(30) Priority: **09.10.81 US 310159**

(43) Date of publication of application: **20.04.83 Bulletin 83/16**

(45) Publication of the grant of the patent: **28.05.86 Bulletin 86/22**

(84) Designated Contracting States: **BE CH DE FR GB IT LI LU NL**

(56) References cited:

**M. NEGWER: "ORGANISCH-CHEMISCHE ARZNEIMITTEL UND IHRE SYNONYMA", 5th edition, vol. II, 1978, Akademie-Verlag, Berlin (DE)**

**CHEMICAL ABSTRACTS, vol. 84, no. 19, May 10, 1976, page 11, abstract no. 130110f, Columbus, Ohio (US) J.L. McCULLOUGH et al.: "Factors affecting human percutaneous penetration of methotrexate and its analogs in vitro"**

**"Martindale, The extra Pharmacopoeia" (1982), pp. 215-9**

(73) Proprietor: **Baylor College of Medicine Texas Medical Center 1200 Moursund Avenue Houston Texas 77030 (US)**

(72) Inventor: **Chan, Raymond Yuen-Fong 16222 Turtleback Road San Diego California, 92127 (US)**
Inventor: **Emery, Jared Maurice 2343 Underwood Houston Texas 77030 (US)**

(74) Representative: **Pennant, Pyers et al Stevens, Hewlett & Perkins 5 Quality Court Chancery Lane London, WC2A 1HZ (GB)**

(56) References cited:

**"Moderne Arzneimittel" (1980), p. 1592, Wissenschaftliche Verlagsgesellschaft Stuttgart (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 077 197 B1

**Description**

Extracapsular cataract extraction has recently become a more popular method of removing cataracts, probably because of its lower incidence of post-operative complications in terms of cystoid macular edema and possible retinal detachment. The advent of an improved extracapsular extraction technique such as phacoemulsification and the requirement of an intact posterior lens capsule for implantation of a wide variety of intraocular lenses have certainly played an important role in influencing such a trend. The only possible disadvantage of extracapsular cataract extraction is the high incidence of posterior lens capsule opacification, which requires additional surgical procedures (posterior capsulotomy or repolishing of the posterior lens capsule) to obtain good vision.

The pathogenesis of posterior lens capsule opacification after extracapsular cataract extraction is known: the remnant lens epithelial cells proliferate on the posterior lens capsule to form abortive lens "fibers" and "bladder" cells (i.e. Elschnig's pearls).

As reported in Contact and Intraocular Lens Medical Journal, Vol. 5, No. 4, Oct/Dec. 1979, pp. 175—178, After-Cataract: Studies of Chemical and Radiation Inhibition, by Roy et al, chemical and radiation means have been attempted to try to find a method associated with extracapsular cataract surgery which would lower the incident of after cataract growth. As reported in this publication the chemicals used (vincristine and vinblastine) were tried to chemically inhibit subcapsular epithelial cells because they had been found to have a direct inhibitory effect on cell mitosis (Goodman, L.S., and Gillman, A: The Pharmacological Basis of Therapeutics, Maximilan, New York 1965, pp. 1373—1376). Vincristine and vinblastine were found to inhibit the corneal wound so that it healed poorly, and because of the deletory effects to the cornea and iris it was the opinion of the authors that these drugs should not be used in further animal studies to try to inhibit subcapsular epithelial perforation. The authors further stated that radiation given the second day after surgery appeared to be the most effective of all dosage schedules, however, they indicate that there is some danger of injury, the authors concluding that it is difficult to say, however, that if one used radiation in humans whether there would be problems or not.

The authors further pointed out that if there were a drug or chemical system that could be found which would inhibit selectively the subcapsular epithelial cells, this might be a useful way to help prevent after cataracts.

We have discovered that subcapsular epithelial growth can be selectively inhibited by instilling methotrexate, retinoic acid or mixtures thereof into the anterior chamber of the eye, following cataract surgery, in initial concentrations to provide a minimal effective dosage of the active agent.

Methotrexate, a known substance, is a cycle-dependent antimetabolite and inhibits the enzyme dihydrofolate reductase thus interfering with the maintenance of intracellular pools of reduced folates. Methotrexate is used therapeutically as an antineoplastic agent, "Martindale, The Extra Pharmacopoeia", (1982), page 215. Retinoic acid is also a known substance and appears to inhibit either cellular division or DNA synthesis (or both). Retinoic acid is used therapeutically as a karatolytic agent, "The Merck Index", 9th Edition, page 1060. We are unaware of any art teaching the instillation of methotrexate, retinoic acid or mixtures thereof, as mitotic inhibitors, into the anterior chamber of the eye in minimal effective dosages at the end of one lens epithelial cell cycle to prevent posterior lens capsule opacification without ocular compromise after extracapsular cataract extraction.

Thus, the present invention provides the use of methotrexate, retinoic acid, or a mixture thereof, for the manufacture of a mitotic inhibitor concentration effective to prevent proliferation of remnant lens epithelial cells when instilled into the anterior chamber of the eye after extracapsular extraction, which mitotic inhibitor composition comprises an osmotically balanced solution containing methotrexate in a minimum concentration of 0.0454 mg per ml of solution, or an osmotically balanced solution containing retinoic acid in a minimum concentration of 0.033 mg per ml of solution, or a mixture thereof.

Methotrexate or retinoic acid, or their mixtures, at precalculated dosages are instilled into the anterior chamber of the eye; preferably immediately after extracapsular cataract extraction which effectively prevent any future proliferation of remnant lens epithelial cells, hence preventing the opacification of the posterior lens capsule. This is accomplished by realising the minimally effective dosage of each active agent, the lens epithelial cell-cycle time, the rate of aqueous humor formation and exit, and the volume of the anterior segment compartments after lens extraction. The initial concentration of the active agent can then be calculated as set forth in the subsequent description.

We have also discovered that another known compound, colchicine, also prevents posterior lens capsule opacification although it has some optic nerve toxocity. In the following description, we have compared the action of colchicine, as a mitotic inhibitor, with the actions of methotrexate and retinoic acid in order to illustrate the advantage of the present invention.

Since all of the structures in contact with the anterior and posterior chambers (corneal endothelium, ciliary epithelium, iris pigment epithelium, muscle, and neural elements) are post-mitotic, cycle-dependant mitotic inhibitors theoretically should affect only the actively dividing lens epithelial cells.

It is essential to determine the necessary initial concentration of each mitotic inhibitor in the anterior segment compartments. This can be

accomplished by realizing the minimal effective dosage of each agent, the lens epithelial cell-cycle time, the rate of aqueous humor formation and exit, and the volume of the anterior segment compartments after lens extraction. The initial concentration can then be calculated from an approximated first-order linear differential equation describing the aqueous humor dynamics:

v=volume of anterior segment compartments
r=rate of aqueous formation (assumed to be equal to aqueous exit in steady state)
C=concentration of the drug
Co=initial concentration
Ct=concentration at time t

Then

$$Ct=C_oe^{-0.00357t}=C_oe^{-4.284}$$

(assuming t=1,200 minutes)

For Methotrexate
$C_t=10^{-6}M=C_oe^{-4.284}$

$\therefore C_o=(10^{-6}M)(e^{4.284})10^{-4}M=\underline{\underline{0.0454\ mg/ml}}$

Retinoic acid
$C_t=10^{-6}M=C_oe^{-4.284}$

$\therefore C_o=(10^{-6}M)(e^{4.284})10^{-4}M=\underline{\underline{0.033\ mg/ml}}$

Colchicine
$C_t=10^{-6}M=C_oe^{-4.284}$

$\therefore C_o=(10^{-6}M)(e^{4.284})72.5\times10^{-6}M=\underline{\underline{0.04\ mg/ml}}$

The carrier solution can be a variety of solutions. It is essentially a sterile osmotically balanced solution, many of which are available on the market, for example, the following;

BSS (Registered Trade Mark) (Balanced Salt Solution) which is a sterile balanced salt solution isotonic to tissues of the eye. Each ml contains sodium chloride 0.64%, potassium chloride 0.075%, calcium chloride 0.048%, magnesium chloride 0.03%, sodium acetate 0.39%, sodium citrate 0.17%, sodium hydroxide and/or hydrochloric acid (to adjust pH) and water for injection.

MIOSTAT (Registered Trade Mark) which is a sterile balanced salt solution of carbachol for intraocular injection. The active ingredient is represented by the chemical structure;

$$NH_2COOCH_2CH_2N(CH_3)_3{}^+Cl^-$$

ZOLYSE (Registered Trade Mark) which is a lyophilized form of crystalline alpha-chymotrypsin, a proteolytic enzyme obtained from the pancreas of the ox. The diluent is a sterile balanced salt solution. A 9 ml vial of diluent contains sodium chloride 0.49%, sodium acetate 0.39%, sodium citrate 0.17%, potassium chloride 0.075%, calcium chloride 0.048%, magnesium chloride 0.03%, hydrochloric acid and/or sodium hydroxide (to adjust pH) and water for injection.

CATARASE (Registered Trade Mark) is a 1:5000 dilution of chymotrypsin in an isotonic, sodium chloride solution.

The diluents used are either BSS for MIOSTAT and ZOLYSE or sterile sodium chloride solution for CATARASE.

Example 1

In this example, extracapsular cataract extraction by phacoemulsification was performed in both eyes of three older primates (*Macaca fascicularis*) and three pigmented rabbits (*Oryctolagus cuniculus*). Methotrexate, retinoic acid, and colchicine was instilled above each of their minimal effective dose into the anterior chamber of one eye of each of the primates and pigmented rabbits immediately following cataract sugery, with the other eye of the same primate and rabbit serving as an operated control.

To determine the effects of the mitotic inhibitors on the remnant epithelial cells, the rabbits were sacrificed six weeks postoperatively despite the lack of any opacifications along the posterior capsules in either the control (operated) or experimental (operated plus mitotic inhibitor instillation). The isolated posterior capsules of the control and experimental animals were processed for electron microscopy and infiltrated in Epon-Araldite. Characteristic light and electron micrographs were taken.

Consistently with all three mitotic inhibitors, light microscopy revealed a homogeneous population of spindle-shaped cells in the angles between the collapsed thick anterior and thin posterior capsules. Uniformly, with all three mitotic inhibitors, any remnant lens epithelial cells along the anterior capsules were non-mitotic, non-pycnotic, and contained contorted interphase nuclei, atrophic mitrochondria, and bloated, irregular rough endoplasmic reticulum. With all three mitotic inhibitors, the spindle cells ultrastructurally were not lens fibers but were transformed arrested lens epithelium that was induced by the mitotic inhibitors to become elongated cells with non-pycnotic nuclei, atrophic mitochondria, and irregular rough endoplasmic reticulum.

Each primate and rabbit was followed with slit-lamp photographs of the posterior lens capsule, endothelial cell counts, and corneal thickness measurements preoperatively, then again at 6 weeks, 4 months, and 8 months postoperatively. Electroretinograms (ERGs) and visual evoked responses (VERs) were recorded preoperatively and then at 6 weeks, 4 months, and 8 months postoperatively.

The most striking clinical result was in the eyes instilled with methotrexate. There was a crystal-clear posterior lens capsule in the experimental eyes; whereas the posterior lens capsule in the control eyes had been completely opacified by the proliferating lens epithelial cells with early developing Elschnig's pearls.

In the eyes instilled with retinoic acid there was a clear central posterior lens capsule with tiny islands of proliferating lens epithelial cells in the far periphery. In the contralateral control eyes there was also a clear central posterior capsule. However, extensive islands of lens epithelial cell proliferation occurred in the mid-periphery.

The posterior lens capsules in the colchicine-injected eyes were clear centrally with peripherial lens epithelial island; however, the control eye showed large islands of epithelial cells with a suggestion of cells breaking through to the posterior clear capsule.

From the sequential ERGs, VERs, endothelial cell counts, and corneal thickness measurements, no ocular toxicity was found in the eyes instilled with methotrexate or retinoic acid, but apparent optic nerve toxicity was seen in the eyes instilled with colchicine.

Example 2

In this example, extracapsular cataract extraction by phacoemulsification was performed in both eyes of four younger primates (approximate human equivalent of 7—10 year old). Methotrexate was instilled in one eye of each of the primates as in Example 1. At two months post-operation, 2 of the 4 posterior lens capsules from the controlled eyes had opacified while all 4 posterior lens capsules instilled with methotrexate remained clear.

From the foregoing examples, and the data obtained, methotrexate and retinoic acid are safe agents to use to prevent posterior lens capsule opacification, with no apparent ocular toxicity or adverse side effects. Colchicine does prevent posterior lens capsule opacification, but apparently there is some optic nerve toxicity associated with its use.

The instillation of methotrexate and retinoic acid in the precalculated dosage as set forth above, provide effective methods to prevent posterior lens capsule opacification without ocular compromise after extracapsular cataract extraction, while instillation of colchicine prevents posterior lens capsule opacification but with some optic nerve toxity.

The use of these mitotic inhibitors minimizes opacification of an intact posterior lens capsule postoperatively, and thereby greatly reduces the complications of cataract surgery, such as cystoid macular edema, retinal detachment, and the corneovitreous touch syndrome.

The present invention, therefore, is well suited and adapted to attain the objects and ends and has the advantages and features mentioned as well as others inherent therein.

**Claim**

The use of methotrexate, retinoic acid or a mixture thereof for the manufacture of a mitotic inhibitor composition effective to prevent proliferation of remnant lens epithelial cells when instilled into the anterior chamber of the eye after extracapsular extraction, which mitotic inhibitor composition comprises an osmotically balanced solution containing methotrexate in a minimum concentration of 0.0454 mg per ml of solution or an osmotically balanced solution containing retinoic acid in a minimum concentration of 0.033 mg per ml of solution, or a mixture thereof.

**Patentanspruch**

Verwendung von Methotrexat, Retinsäure oder einer Mischung davon für die Herstellung einer mitotischen Inhibitorzusammensetzung zur Verhinderung von Wucherungen restlicher Linsen-Epithelial-Zellen beim Einsetzen in die vordere Augenkammer nach extra-kapsulärer Extraktion, dadurch gekennzeichnet, dass die mitotische Inhibitorzusammensetzung eine osmotisch ausgeglichene Lösung, enthaltend Methotrexat, in einer Minimalkonzentration von 0,0454 mg/ml Lösung oder eine osmotisch ausgeglichene Lösung, enthaltend Retinsäure, in einer Minimalkonzentration von 0,033 mg/ml Lösung, oder eine Mischung davon enthält.

**Revendication**

Utilisation du méthotrexate, de l'acide rétinoïque ou d'un mélange de ces substances, pour la préparàtion d'une composition d'inhibiteur mitotique efficace pour empêcher la prolifération de cellules épithéliales restantes de cristallin lorsqu'elle est instillée dans la chambre antérieure de l'oeil, après une extraction extra-capsulaire, ladite composition d'inhibiteur mitotique comprenant une solution osmotiquement équilibrée contenant du méthotrexate en une concentration minimale de 0,0454 mg/ml de solution, ou une solution osmotiquement équilibrée contenant de l'acide rétinoïque en une concentration minimale de 0,033 mg/ml de solution, ou un mélange de ces solutions.